# EUROPEAN PATENT SPECIFICATION

(11) **EP 2 334 306 B1**
(45) Date of publication and mention of the grant of the patent: **02.04.2014**
(21) Application number: 09810593.5
(22) Date of filing: 27.08.2009
(51) Int. Cl.: A61K 31/43, A61K 31/7036, A61K 45/06, A61K 38/46

(54) **METHODS OF MODULATING GASTROINTESTINAL TRACT FLORA LEVELS WITH ALKALINE PHOSPHATASE**
VERFAHREN ZUR MODULATION DER MAGEN-DARM-FLORA MIT ALKALISCHER PHOSPHATASE
PROCÉDÉS DE MODULATION DES TAUX DE FLORE DU TRACTUS GASTRO-INTESTINAL AVEC UNE PHOSPHATASE ALCALINE

(30) Priority: 29.08.2008 US 93129 P
(43) Date of publication of application: 22.06.2011
(73) Proprietor: The General Hospital Corporation, Boston, MA 02114 (US)
(72) Inventor: HODIN, Richard A., Newton Massachusetts 02459 (US); MALO, Madhu S., Watertown Massachusetts 02474 (US)
(74) Representative: Coehn, Markus
(86) International application number: PCT/US2009/055216
(87) International publication number: WO 2010/025267

(56) References cited:
- EP-A1- 1 952 823
- WO-A1-2005/074978
- WO-A2-2004/112494
- RANNA A ROZENFELD ET AL.: 'The Role of Intestinal Flora in Endotoxin (LPS)-Ind uced Gut Injury and Group II Phospholipase A2 (PLA2-II) Activation in the Sm all Intestine.' PEDIATRIC RESEARCH. vol. 45, no. 4, April 1999, page 45A, XP008144636
- SHIPRA VAISHNAVA ET AL.: 'Alkaline Phosphatase: Keeping the Peace at the Gut Epithelial Surface.' CELL HOST MICROBE. vol. 2, no. 6, 12 December 2007, pages 365 - 367, XP008145216
- JENNIFER M. BATES ET AL.: 'Intestinal alkaline phosphatase detoxifies lipo polysaccharide and prevents inflammation in zebrafish in response to the gut microbiota.' CELL HOST MICROBE. vol. 2, no. 6, 12 December 2007, pages 371 - 382, XP008145630

## Description

### TECHNICAL FIELD

This invention relates to methods of modulating levels of gastrointestinal tract flora with alkaline phosphatase.

### BACKGROUND

Through millions of years of evolution, metazoans have developed mechanisms that maintain a mutually beneficial symbiotic relationship with commensal microbiota. Intestinal microbes play a pivotal role in maintaining human health and wellbeing (Marchesi and Shanahan, Curr Opin Infect Dis. 20:508, 2007; Blaut and Clavel, J Nutr. 137:751S, 2007; Turnbaugh et al., Nature, 444:1027, 2006; Dethlefsen et al., Trends Ecol Evol, 21:517, 2006; Lupp and Finlay, Curr Biol, 15:R235, 2005; Backhed et al., Science, 307:1915, 2005; Mai and Morris, Jr., J Nutr, 134:459, 2004; Falk et al., Microbiol Mol Biol Rev, 62:1157, 1998). Mucosa-associated bacteria, in conjunction with the mucus-layer and the epithelium, provide a direct selective barrier between the gut lumen and systemic sites, allowing the absorption of nutrients, water, and electrolytes, while inhibiting the translocation of pathogenic microbes or toxins. The microbiota synthesizes vitamins, detoxifies toxins, and also provides additional energy for the epithelial cells by fermentation of otherwise non-digestible food components, such as fiber and starch. Commensal organisms also participate in the maturation and maintenance of the gastrointestinal tract immune system, keeping the intestinal epithelia in a state of 'physiological inflammation' that appears to be critical for a rapid response against potentially harmful bacteria.

The human gastrointestinal tract harbors approximately 10¹⁴ bacteria that are composed of between 300 and 1,000 different species (Kullberg, Nature, 453:602, 2008; Baba et al., J Leukoc Biol, 84:468, 2008). A reduction in the normal levels and function of flora that occur naturally in the gastrointestinal tract of animals and in humans can cause various symptoms. Dysbiosis, defined as dysregulation of the normal homeostasis of the intestinal microbiota, has been implicated in the pathogenesis of myriad disease conditions, including, but not limited to, antibiotic-associated diarrhea (AAD), *Clostridium difficile*-associated disease (CDAD), acquired immunodeficiency syndrome (AIDS), hypothyroidism, food poisoning, obesity, inflammatory bowel disease (IBD), irritable bowel syndrome (IBS), and colorectal carcinoma (Dethlefsen et al., Trends Ecol Evol, 21:517, 2006; Schroeder, Am Fam Physician, 71:921, 2005; Hurley and Nguyen, Arch Intern Med, 162:2177, 2002; Bartlett, N Engl J Med, 346:334, 2002; McFarland, Dig Dis, 16:292, 1998; Wistrom et al., J Antimicrob Chemother, 47:43, 2001; Marteau et al., Aliment Pharmacol Ther, 20 Suppl 4:18, 2004; Seksik et al., Gut, 52:237, 2003; Kleessen, Scand J Gastroenterol, 37:1034, 2002; Brenchley et al., Nat Med, 12:1365, 2006; Mai and Morris, J Nutr, 134:459, 2004; Seksik et al., Gut, 52:237, 2003; Marteau et al., Aliment Pharmacol Ther, 20 Suppl 4:18, 2004; Kleessen et al., Scand J Gastroenterol, 37:1034, 2002; Pool-Zobel et al., Br J Nutr, 87 Suppl 2:S273, 2002). Epidemiological studies have also linked altered composition of the intestinal microbiota with the development of rheumatoid arthritis, eczema, and other allergic diseases (Lupp and Finlay, Curr Biol, 15:R235, 2005; Toivanen, Ann Rheum Dis, 62:807, 2003) and aberrant microbiota during childhood may predispose one to the development of inflammatory gut diseases and diarrhea (Juntunen et al., Clin Diagn Lab Immunol, 8:293, 2001; Arvola et al., Pediatrics, 104:e64, 1999).

However, the fundamental mechanisms that govern the normal homeostatic number and composition of the intestinal microbiota remain poorly understood. Factors that have been implicated in influencing the gastrointestinal tract flora include antimicrobial peptides, age, nutrition, immune status, luminal pH, fecal transit time, interactions among components of the flora, available fermentable materials, medications, stress, general living conditions, etc. (Collins and Gibson, Am J Clin Nutr, 69:1052S, 1999; Gibson and Fuller, J Nutr, 130:391S, 2000).

WO 2005/074978 A1 discloses a food formulation e.g. milk, including alkaline phosphatase.

EP 1 952823 A1 discloses a medicament for oral administration including alkaline phosphatase.

WO 2005/074978 A1 discloses a food or beverage e.g. milk, including alkaline phosphatase e.g. calf intestinal alkaline phosphatase.

SHIPRA VAISHNAVA ET AL.: "Alkaline Phosphatase: Keeping the Peace at the Gut Epithelial Surface.", CELL HOST MICROBE., vol. 2, no. 6, 2007, pages 365-367, discloses that intestinal alkaline phosphatase detoxifies LPS and promotes mucosal tolerance to commensal bacteria.

JENNIFER M. BATES ET AL.: "Intestinal alkaline phosphatase detoxifies lipopolysaccharide and prevents inflammation in zebrafish in response to the gut microbiota.", CELL HOST MICROBE., vol. 2, no. 6, 2007, pages 371- 382, discloses that intestinal alkaline phosphatase detoxifies LPS.

### SUMMARY

This invention is based, at least in part, on the discovery that administering, e.g., orally, an alkaline phosphatase, e.g., an intestinal alkaline phosphatase, to a subject in a therapeutically effective amount, can rapidly restore gastrointestinal tract microbiota to normal levels and/or function, e.g., lost during antimicrobial treatment, and can thus safely and easily reduce one or more symptoms such as diarrhea, e.g., antibiotic-associated diarrhea (AAD), with little or no side effects. The new methods are effective for a variety of subjects including humans and animals, such as laboratory animals, e.g., mice, rats, rabbits, or monkeys, or domesticated and farm animals, e.g., cats, dogs, goats, sheep, pigs, cows, horses, and birds, e.g., chickens and turkeys.

The invention is further specified in the claims. In one aspect, the patent features methods of modulating gastrointestinal tract flora levels in a subject, such as a human or animal subject. The methods include administering to the subject an amount of an alkaline phosphatase effective to increase the number of commensal bacteria in the gastrointestinal tract, decrease the number of pathogenic bacteria in the gastrointestinal tract, or both increase the number of commensal bacteria and decrease the number of pathogenic bacteria in the gastrointestinal tract of the subject.

In another general aspect, the patent features methods of reducing one or more symptoms of antibiotic-associated diarrhea (AAD) or *Clostridium difficile*-associated disease (CDAD) in a subject, the methods including administering, e.g., orally, to the subject an amount of an alkaline phosphatase, e.g., intestinal alkaline phosphatase, effective to reduce one or more symptoms of AAD or CDAD.

In another aspect, the patent features methods of modulating gastrointestinal tract flora levels in a subject by administering alkaline phosphatase at or at about the same time as a diagnosis of aberrant levels of gastrointestinal tract flora (e.g., therapeutically), when aberrant levels of gastrointestinal tract flora are associated with a disorder (e.g., antibiotic-associated diarrhea (AAD), *Clostridium difficile*-associated disease (CDAD), acquired immunodeficiency syndrome (AIDS), hypothyroidism, colorectal carcinoma, obesity, rheumatoid arthritis, eczema, allergy, radiotherapy or chemotherapy treatment, stress, and food poisoning), when aberrant levels of gastrointestinal tract flora are anticipated (e.g., prophylactically), or at or at about the same time as an antibiotic (e.g., Amikacin, Gentamicin, Kanamycin, Neomycin, Netilmicin, Streptomycin, Tobramycin, Paromomycin, Geldanamycin, Herbimycin, Loracarbef, Ertapenem, Doripenem, Imipenem/Cilastatin, Meropenem, Cefadroxil, Cefazolin, Cefalotin, Cefalexin, Cefaclor, Cefamandole, Cefoxitin, Cefprozil, Cefuroxime, Cefixime, Cefdinir, Cefditoren, Cefoperazone, Cefotaxime, Cefpodoxime, Ceftazidime, Ceftibuten, Ceftizoxime, Ceftriaxone, Cefepime, Ceftobiprole, Teicoplanin, Vancomycin, Azithromycin, Clarithromycin, Dirithromycin, Erythromycin, Roxithromycin, Troleandomycin, Telithromycin, Spectinomycin, Aztreonam, Amoxicillin, Ampicillin, Azlocillin, Carbenicillin, Cloxacillin, Dicloxacillin, Flucloxacillin, Mezlocillin, Meticillin, Nafcillin, Oxacillin, Penicillin, Piperacillin, Ticarcillin, Bacitracin, Colistin, Polymyxin B, Ciprofloxacin, Enoxacin, Gatifloxacin, Levofloxacin, Lomefloxacin, Moxifloxacin, Norfloxacin, Ofloxacin, Trovafloxacin, Grepafloxacin, Sparfloxacin, Mafenide, Prontosil, Sulfacetamide, Sulfamethizole, Sulfanilimide, Sulfasalazine, Sulfisoxazole, Trimethoprim, Trimethoprim-Sulfamethoxazole (Co-trimoxazole), Demeclocycline, Doxycycline, Minocycline, Oxytetracycline, Tetracycline, Arsphenamine, Chloramphenicol, Clindamycin, Lincomycin, Ethambutol, Fosfomycin, Fusidic acid, Furazolidone, Isoniazid, Linezolid, Metronidazole, Mupirocin, Nitrofurantoin, Platensimycin, Pyrazinamide, Quinupristin/Dalfopristin, Rifampin, Thiamphenicol, Tinidazole, Cephalosporin, Teicoplatin, Augmentin, Cephalexin, Rifamycin, Rifaximin, Cephamandole, Ketoconazole, Latamoxef, or Cefinenoxime) is administered.

In these methods, the pathogenic bacteria can be, for example, *Acetobacter aurantius, Acinetobacter baumannii*, *Actinomyces israelii*, *Agrobacterium radiobacter*, *Agrobacterium tumefaciens*; *Azorhizobium caulinodans, Azotobacter vinelandii, Anaplasma, Anaplasma phagocytophilum, Anaplasma marginale, Bacillus anthracis, Bacillus brevis, Bacillus cereus, Bacillus fusiformis, Bacillus licheniformis, Bacillus megaterium, Bacillus mycoides, Bacillus stearothermophilus, Bacillus subtilis, Bacteroides fragilis, Bacteroides gingivalis, Bartonella henselae, Bartonella quintana, Bordetella bronchiseptica, Bordetella pertussis, Borrelia burgdorferi, Brucella abortus*, *Brucella melitensis, Brucella suis, Burkholderia mallei*, *Burkholderia pseudomallei, Burkholderia cepacia, Calymmatobacterium granulomatis, Campylobacter coli, Campylobacter fetus, Campylobacter jejuni, Campylobacter pylori, Chlamydia trachomatis, Chlamydophila pneumoniae, Chlamydophila psittaci, Clostridium botulinum, Clostridium difficile, Clostridium perfringens, Clostridium tetani, Corynebacterium diphtheriae, Corynebacterium fusiforme, Coxiella burnetii, Ehrlichia chaffeensis, Enterobacter cloacae, Enterococcus avium, Enterococcus durans, Enterococcus faecalis, Enterococcus faecium, Enterococcus galllinarum, Enterococcus maloratus,* enteropathogenic *Escherichia coli, Francisella tularensis, Fusobacterium nucleatum, Gardnerella vaginalis, Haemophilus ducreyi, Haemophilus influenzae, Haemophilus parainfluenzae, Haemophilus pertussis, Haemophilus vaginalis, Helicobacter pylori, Klebsiella pneumoniae, Lactobacillus acidophilus, Lactobacillus casei, Lactococcus lactis, Legionella pneumophila, Listeria monocytogenes, Methanobacterium extroquens, Microbacterium multiforme, Micrococcus luteus, Moraxella catarrhalis, Mycobacterium avium, Mycobacterium bovis, Mycobacterium diphtheriae, Mycobacterium intracellulare, Mycobacterium leprae, Mycobacterium lepraemurium, Mycobacterium phlei, Mycobacterium smegmatis, Mycobacterium tuberculosis, Mycoplasma fermentans, Mycoplasma genitalium, Mycoplasma hominis, Mycoplasma penetrans, Mycoplasma pneumoniae, Neisseria gonorrhoeae, Neisseria meningitides, Pasteurella multocida, Pasteurella tularensis, Peptostreptococcus, Porphyromonas gingivalis, Prevotella melaninogenica, Pseudomonas aeruginosa, Rhizobium radiobacter, Rickettsia prowazekii, Rickettsia psittaci, Rickettsia quintana, Rickettsia rickettsii, Rickettsia trachomae, Rochalimaea henselae, Rochalimaea quintana, Rothia dentocariosa, Salmonella enteritidis, Salmonella typhi, Salmonella typhimurium, Serratia marcescens, Shigella dysenteriae, Staphylococcus aureus, Staphylococcus epidermidis, Stenotrophomonas maltophilia, Streptococcus agalactiae, Streptococcus avium, Streptococcus bovis, Streptococcus cricetus, Streptococcus faceium, Streptococcus faecalis, Streptococcusferus, Streptococcus gallinarum. Streptococcus lactis, Streptococcus mitior, Streptococcus mitis, Streptococcus mutans, Streptococcus oralis, Streptococcus pneumoniae, Streptococcus pyogenes, Streptococcus rattus, Streptococcus salivarius, Streptococcus sanguis, Streptococcus sobrinus, Treponema pallidum, Treponema denticola, Vibrio cholerae, Vibrio comma. Vibrio parahaemolyticus, Vibrio vulnificus, Wolbachia, Yersinia enterocolitica, Yersinia pestis,* or *Yersinia pseudotuberculosis.*

In these methods, the amount of alkaline phosphatase administered can be, for example, from about 1 to about 10,000, e.g., 1 to 200, 200 to 500, 500 to 1,000, 1,000 to 5,000, or 5,000 to 10,000, units per kilogram of body weight. Higher doses, e.g., 10,000 to 50,000 units per kilogram of body weight, are also possible. These dosages can be administered as a single bolus or as an infusion over one or more hours or days. Further, alkaline phosphatase can be administered at the same time and length as antibiotic treatment or every day for subjects who have, or at risk of developing, acquired immunodeficiency syndrome (AIDS), hypothyroidism, colorectal carcinoma, rheumatoid arthritis, eczema, allergy, stress, food poisoning, or undergoing radiotherapy or chemotherapy treatment.

In yet another aspect, the patent features beverage and food products comprising an alkaline phosphatase (e.g., intestinal alkaline phosphatase) effective to modulate gastrointestinal tract flora levels in a subject. The beverage products can contain from 1 to 10,000, e.g., 1 to 200, 200 to 500, 500 to 1,000, 1,000 to 5,000, or 5,000 to 10,000, units per milliliter. The food products can contain from 1 to 10,000, e.g., 1 to 200, 200 to 500, 500 to 1,000, 1,000 to 5,000, or 5,000 to 10,000, units per gram.

As used herein, the term "antibiotic-associated diarrhea (AAD)" means diarrhea following antimicrobial treatment due to the changes in the composition and function of the intestinal flora. AAD can, but need not, be accompanied by a *Clostridium difficile* infection.

The invention provides several advantages. The treatment of AAD in human subjects with Metronidazole^{®} and Vancomycin^{®} can lead to the development of serious side-effects including seizure, peripheral neuropathy, hepatotoxicity, ototoxicity, and nephrotoxicity. The prophylactic and therapeutic methods described herein using an alkaline phosphatase are effective in restoring normal gastrointestinal tract flora and have minimal, if any, side effects. When used for treating domestic animals, particularly animals used in commercial farming and food production, the new methods provide cost savings triggered by avoiding AAD-associated side effects, which allow a better feed utilization, faster weight gain, and shorter time to market the treated animals.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. All publications, patent applications, patents, and other references mentioned herein are incorporated by reference in their entirety. In case of conflict, the present specification, including definitions, will control. In addition, the materials, methods, and examples are illustrative only and not intended to be limiting.

Other features, objects, and advantages of the invention will be apparent from the detailed description, and from the claims.

### DESCRIPTION OF DRAWINGS

FIG. 1A is an image showing growth of Gram-negative bacteria from the stools of wild type (WT) mice on MacConkey (Mac) agar plates (0.01 mg stool plated).
FIG. 1B is an image showing no growth of Gram-negative bacteria from the stools of intestinal alkaline phosphatase (IAP) knockout (KO) mice (IAP-KO) on MacConkey agar plates (10 mg stool plated).
FIG. 1C is a bar graph depicting total count of bacteria from the stools of WT and KO mice (n = 7) grown in aerobic conditions on Luria Bertani (LB), Brain Heart Infusion (BHI), MacConkey (Mac), and Brucella agar plates.
FIG. 1D is a bar graph depicting total count of bacteria from the stools of WT and KO mice (n = 7) grown in anaerobic conditions on Brucella agar plates.
FIG. 2A is a bar graph depicting Terminal Restriction Fragment Length Polymorphism (TRFLP) peaks per sample of restriction digests (*Dpn* II, *Hae* III, *Hpa* II, and all combined) of bacterial 16S rRNA obtained from the stools of WT and IAP-KO mice.
FIG. 2B is a scatter plot with TRFLP peaks ordinated using multidimentional scaling (MDS) to show the similarity in the TRFLP profiles of individual as well as combined samples *of Dpn* II, *Hae* III, and *Hpa* II restriction digests of bacterial 16S rRNA obtained from WT and IAP-KO mice stool samples. *, significant (p < 0.05).

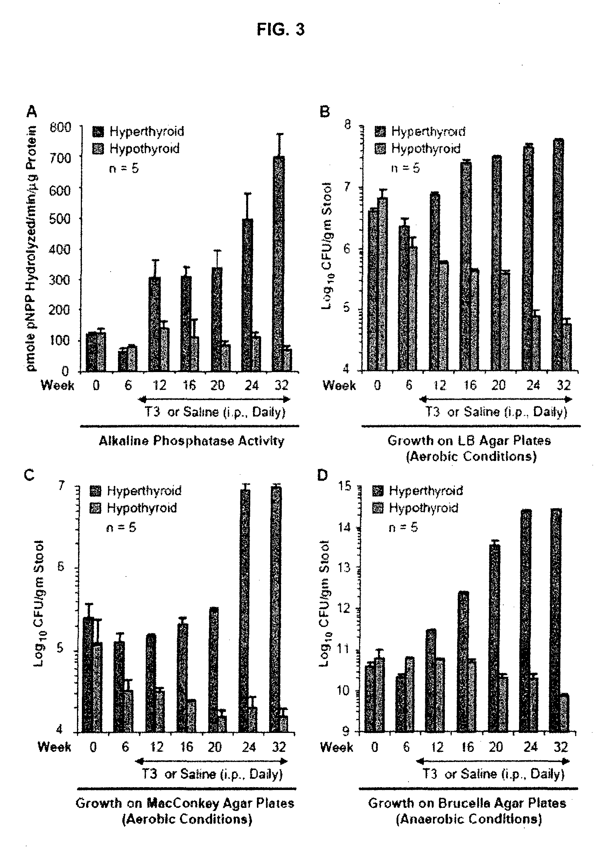

FIGs. 3A, 3B, 3C, and 3D are a series of four bar graphs showing alkaline phosphatase activity and bacterial counts in the stools of hyper- and hypothyroid mice. (3A) Alkaline phosphatase activity was measured in the stools of hyper- and hypothyroid mice. Specific activity of IAP was expressed as mean pmole p-nitrophenyl phosphate (pNPP) hydrolyzed/min/µg protein +/- SEM. (3B) Total CFU from the stools of hyper-and hypothyroid mice grown on LB agar plates under aerobic conditions. (3C) Growth of Gram-negative bacteria from the stools of hyper- and hypothyroid mice grown on MacConkey agar plates under aerobic conditions. (3D) Total CFU from the stools of hyper- and hypothyroid mice grown on Brucella agar plates under anaerobic conditions.
FIG. 4A is a line graph showing that commensal *E. coli* fail to colonize IAP-KO mice.
FIG. 4B is a bar graph depicting that oral supplementation of IAP favors survival of *E*. *coli* in IAP-KO mice. Unpaired Student's *t*-Test or Fisher's Exact Test were used to calculate the statistical significance. *, significant.
FIG. 4C is a bar graph showing temporal enhancement of *E. coli* colonization in WT mice by IAP following administration of streptomycin and ciprofloxacin. Unpaired Student's *t*-Test or Fisher's Exact Test were used to calculate the statistical significance. *, significant.
FIG. 5 is a line graph showing the relative stability of calf intestinal alkaline phosphatase (cIAP) activity in autoclaved tap water over a period of 120 hours.
FIG. 6 is a bar graph showing that mice receiving higher amounts of cIAP in drinking water have increased IAP activity in their stools.
FIGs. 7A and 7B are two bar graphs showing that oral IAP supplementation enhances the restoration of antibiotic-associated loss of gut flora (7A) and inhibits *Salmonella typhimurium* infection in WT mice (7B).

### DETAILED DESCRIPTION

The gastrointestinal tract flora consists of microorganisms that normally live in the digestive tract of animals. Research suggests that the relationship between gut flora and humans is not merely commensal, but rather a mutualistic, symbiotic relationship (Sears, Anaerobe, 11: 247, 2005). The microorganisms perform a host of useful functions, including preventing growth of harmful species (Guarner and Malagelada, Lancet, 361:512, 2003), fermenting unused energy substrates, training the immune system, regulating the development of the gut, producing vitamins for the host (such as biotin and vitamin K), and producing hormones to direct the host to store fats. However, in certain conditions, some bacterial species are thought to be capable of causing disease.

Provided herein are methods for modulating gastrointestinal tract flora levels in a subject. The patent relates to methods of treating subjects with reduced levels and/or function of gastrointestinal tract flora by administering an alkaline phosphatase in an amount effective to increase or preserve the number of commensal bacteria and composition of intestinal microbiota. This patent also relates to methods of treating subjects with an alkaline phosphatase to treat infections by pathogenic bacteria and/or inhibit the growth or decrease the number of pathogenic bacteria in the gastrointestinal tract. The methods described can be used to treat symptoms associated with reduced levels of commensal bacteria and/or function of gastrointestinal tract flora, e.g., antibiotic-associated diarrhea (AAD), *Clostridium difficile*-associated disease (CDAD), acquired immunodeficiency syndrome (AIDS), hypothyroidism, colorectal carcinoma, obesity, rheumatoid arthritis, eczema, allergy, radiotherapy, chemotherapy, stress, and food poisoning.

### Diagnosing Loss of Function And/Or Level of Normal Gut Flora

The loss of function and/or level of normal gastrointestinal tract flora can be generally diagnosed by the typically accompanying diarrhea and other symptoms described below. For example, antibiotic-associated diarrhea (AAD), which is otherwise unexplained diarrhea that occurs in association with the administration of antibiotics, can be diagnosed as discussed below. The frequency of this complication varies among antibacterial agents. Typically no disease causing pathogens are identified and the diarrhea develops due to changes in the composition and function of the intestinal flora. The diagnosis of AAD should be considered in any patient recently treated with antibiotics and presenting with new onset of diarrhea. Exposure up to 8 weeks before onset of symptoms to any antimicrobial or antifungal agent should be considered as a possible cause of AAD.

Clinical presentation, laboratory data, imaging, and endoscopic examinations are all useful to making a proper diagnosis of AAD. Atypical, subtle presentations, especially in ambulatory patients with a remote and brief antibiotic exposure, require high suspicion. Leukocytosis, fecal leukocytes, and fecal occult blood are supportive of the diagnosis, but are not always present. An increase in the abundance of opportunistic pathogens such as C. *difficile* is frequently identified in patients with signs and symptoms of colitis and it is the most frequent cause of diarrhea in hospitalized patients.

The cornerstone of the diagnosis of *C*. *difficile* colitis is identification of *C*. *difficile* toxins in the stool. Culture assays are considered to be the "gold standard," based on the demonstration of toxin B cytopathic effects on cell culture monolayers. This test carries great specificity and sensitivity in detecting minimal toxin concentrations. Unfortunately, cell culture tests are quite expensive, time consuming, and rarely used in clinical practice.

Another method for detecting and diagnosing *C*. *difficile* colitis is the enzyme-linked immunosorbent assay (ELISA), based on toxin detection in the stool. Most commercially available methods detect both A and B toxins, obviating the problems of missing certain *C*. *difficile* strains that produce only toxin B. The ELISA is fast, relatively inexpensive, and has excellent specificity; its sensitivity, however, is limited to 75% to 85%. Serial stool determinations on different days are suggested in suspected cases with initial negative results.

Another diagnostic test for *C*. *difficile,* which is less frequently used due to a lack of specificity, is the latex agglutination test. The test is based on the detection of the enzyme glutamate dehydrogenase rather than on *C*. *difficile* toxin. Nontoxigenic strains of C. *difficile* as well as other colonic organisms may produce this enzyme.

Very often, nonspecific findings of colitis such as edema, erythema, and loss of vascular pattern are the only findings. In cases of pseudomembranous colitis, endoscopy is diagnostic as it may reveal typical raised, yellow nodules over areas of normal mucosa or minimal erythema. In more severe cases, coalescent nodules forming extensive areas of pseudomembranes over a background of inflammation and ulcerations are found. In most cases, pseudomembranes are distributed throughout the colon and are readily identified within the reach of the sigmoidoscope. In up to one third of cases, the pseudomembranes can be confined to the right colon, making colonoscopy necessary when less extensive procedures do not confirm a diagnosis that is strongly suspected. When pseudomembranes are not grossly visible, mucosal biopsies may show characteristic findings.

Approximately 10¹⁴ bacteria can be found in a normal human gastrointestinal tract (Kullberg, Nature, 453:602, 2008; Baba et al., J Leukoc Biol, 84:468, 2008). Levels of gastrointestinal tract flora can be determined by measuring the number of bacteria in a stool sample. Normal levels of bacteria found in stool samples range from 10⁹ to 10¹³ CFU/gram of dry stool (Chen et al, J Dairy Sci, 82:2308, 1999). Alternatively, normal levels of bacteria found in stool samples can be determined by examining the stool samples from at least 20 subjects who do not have gastrointestinal problems associated with altered levels of flora (e.g., caused by AAD, CDAD, AIDS, hypothyroidism, food poisoning, obesity, IBD, IBS, colorectal carcinoma, obesity, rheumatoid arthritis, eczema, allergy, undergoing radiotherapy or chemotherapy treatment, or under stress).

### General Methodology

The methods can be used as a treatment for any diagnosed reduction in the level and/or function of normal gut flora, such as for diagnosed AAD, or can be used prophylactically with any administration of an antibiotic in all patients or in certain patient populations, e.g., in patient populations at higher risk for disorders such as AAD, e.g., those patients over 60 years of age, those patients with previous bouts of AAD, and patients who have a compromised immune system (e.g., acquired immunodeficiency syndrome (AIDS) patients and others). Other populations at higher risk are patients who have had abdominal surgery, are prescribed a prolonged use of antibiotics, or who have been in the hospital for more than 2, 3, or 4 weeks. The methods are simple and effective and involve administering an effective amount of an alkaline phosphatase.

Alkaline phosphatases are hydrolase enzymes responsible for removing phosphate groups from many types of molecules, including nucleotides, proteins, and alkaloids. The process of removing the phosphate group is called dephosphorylation. As the name suggests, alkaline phosphatases are most effective in an alkaline environment, with optimal enzyme activity around pH 10. In humans, alkaline phosphatases are present in all tissues throughout the body, but they are particularly concentrated in the liver, kidney, placenta, in growing bone, and in the bile duct. Alkaline phosphatase is released into the blood during injury and during such normal activities as bone growth and pregnancy. Levels of alkaline phosphatase can be measured in routine blood tests.

Abnormally high blood levels of alkaline phosphatase may indicate disease in bone or liver, bile duct obstruction, or certain malignancies. For example, the enzyme is often elevated in the leukemic cells in chronic myelogenous leukemia. Abnormally low levels of alkaline phosphatase indicate a genetic condition called hypophosphatasia, which results in bone deformities.

Intestinal alkaline phosphatase (IAP) is a brush-border enzyme that is expressed exclusively in villus-associated enterocytes of the small intestine and has the primary function of hydrolyzing monophosphate esters, splitting cholesterol and long chain fatty acids, and is associated with the ability to assimilate calcium. It has been shown that IAP is capable of detoxifying lipopolysaccharides (LPS), likely through dephosphorylation of the Lipid-A moiety, the primary source of its endotoxic effects (Beumer et al., J Pharmacol Exp Ther, 307:737, 2003).

### Methods of Administration

In general, alkaline phosphatase is mixed with standard pharmaceutically acceptable excipients, and can be administered orally, intravenously, or rectally. Alkaline phosphatase can be intestinal alkaline phosphatase, calf intestinal alkaline phosphatase, bovine IAP, chicken IAP, goat IAP, bacterial alkaline phosphatase, fungal alkaline phosphatase, shrimp alkaline phosphatase, placental alkaline phosphatase, secretable placental alkaline phosphatase, placental-like alkaline phosphatase, bone alkaline phosphatase, liver alkaline phosphatase, kidney alkaline phosphatase, germ line alkaline phosphatase, tissue non-specific alkaline phosphatase, modified IAP, recombinant IAP, any peptide comprising alkaline phosphatase activity. Alkaline phosphatase is available in standard dosage units, and can be administered in dosages from about 1 to 10,000 units per kg body weight, or even higher. These dosages can be administered as a single bolus or as an infusion over one or more hours or days. Further, alkaline phosphatase can be administered at the same time and length as antibiotic treatment or every day for subjects who have, or at risk of developing, acquired immunodeficiency syndrome (AIDS), hypothyroidism, colorectal carcinoma, obesity, rheumatoid arthritis, eczema, allergy, stress, food poisoning, or undergoing radiotherapy or chemotherapy treatment. Testing has shown that this range of dosage is non-toxic and non-immunogenic.

As far as efficacy is concerned, oral administration is suitable. For instance, alkaline phosphatase can be administered in beverages (e.g., milk, juice, soda, and other flavored liquids), yogurt (e,g., plain or flavored yogurt, yogurt drinks, and frozen yogurt), and foods (e.g., cereal, cereal bars, energy bars, and ice cream). The liquid or powder form of alkaline phosphatase can be taken by adding an additive such as a sweetener if necessary. A variety of well-known substances such as bonding agents, forming agents, lubricants, brightening agents, sweeteners, and zests, can be used. One is not restricted by these additional methods. Additionally, various methods can be used when combining alkaline phosphatase with conventional beverages and foods. In such cases, the amount of alkaline phosphatase used can be appropriately adjusted according to an individual's eating and drinking habits.

The methods of the present invention can be used to treat reduced levels of commensal bacteria caused by a variety of disorders. These disorders include antibiotic-associated diarrhea (AAD), *Clostridium difficile*-associated disease (CDAD), acquired immunodeficiency syndrome (AIDS), hypothyroidism, colorectal carcinoma, obesity, rheumatoid arthritis, eczema, allergy, radiotherapy, chemotherapy, stress, and food poisoning.

### Antibiotic-Associated Diarrhea (AAD) and Clostridium difficile-Associated Disease (CDAD)

A reduction in the normal levels and function of flora that occur naturally in the gastrointestinal tract of various animals and in humans can cause various symptoms, including diarrhea. For example, antibiotic-associated diarrhea (AAD) is the most common cause of diarrhea in hospitalized patients, representing an important source of morbidity, mortality, and cost. The term AAD refers to a benign, self-limited diarrhea, following the use of antimicrobials. Typically no disease causing pathogens are identified and the diarrhea develops due to changes in the composition and function of the intestinal flora. The occurrence of AAD varies greatly and is influenced by multiple factors including nosocomial outbreaks, patterns of antimicrobial use, and individual susceptibility.

Although no infectious agent is found in most cases of AAD, *Clostridium difficile* is frequently identified as the infectious agent in patients with signs and symptoms of colitis and it is the most frequent cause of diarrhea in hospitalized patients. This disease is referred to as *Clostridium difficile*-associated disease (CDAD). It is estimated that 10% to 15% of all hospitalized patients treated with antibiotics will develop AAD and twice as many will become asymptomatic carriers. Risk factors for the development include compromised immune status, advanced age, abdominal surgery, comorbidity, type and prolonged use of antibiotics, and the length of hospitalization. For example, infection rates for *C*. *diffcile* are reported to be around 10% after 2 weeks of hospitalization, but may reach 50% after 4 or more weeks (McFarland, Dig Dis, 16:292, 1998). In addition, persons over 60 years of age have an incidence of *Clostridium difficile* infection that is 20 to 100 times higher than patients 10 to 20 years of age (Bartlett, N Eng J Med, 346:334, 2002).

All groups of antibiotics can cause AAD, leading to a wide range of clinical manifestations, from an asymptomatic carrier state to severe pseudomembranous colitis. Those antibiotics with broad-spectrum coverage, in particular cephalosporins, extended-coverage penicillins, and clindamycin are the most common culprits (Wistrom et al., J Antimicrob Chemother, 47:43, 2001).

In most cases of AAD, patients respond to supportive measures and withdrawal of antibiotics. However, in patients with severe and persistent symptoms or the diagnosis of CDAD, antibiotic therapy with Metronidazole^{®} or in more severe cases Vancomycin^{®} is available, but relapses are common. Also, Metronidazole® and Vancomycin® treatment can cause serious side-effects, including, seizure, peripheral neuropathy, hepatotoxicity, ototoxicity, and nephrotoxicity. Other antimicrobial agents, in particular bacitracin and teicoplatin, have been used with some success in the past. However, their use is limited to situations in which Metronidazole^{®} or Vancomycin^{®} cannot be used or have failed (Young et al., Gastroenterology, 89:1038, 1985). The methods described herein provide an effective alternative therapy with no known side effects that can be used regardless of the course of the AAD.

### Acquired Immunodeficiency Syndrome (AIDS)

Gastrointestinal disorders are among the most frequent complaints in patients with human immunodeficiency virus 1 (HIV-1) or human immunodeficiency virus 2 (HIV-2) acquired immunodeficiency syndrome (AIDS). The majority of AIDS subjects have marked gastrointestinal symptoms during the course of their illness. Gastrointestinal manifestations of HIV disease include diarrhea, dysphagia, odynophagia, nausea, vomiting, weight loss, abdominal pain, anorectal disease, jaundice, hepatomegaly, gastrointestinal tract bleeding, and gastrointestinal tumors (e.g., Kaposi's sarcoma and non-Hodgkin's lymphoma). Progressive immunocompromisation is associated with increasing prevalence of gastrointestinal symptoms and remains the common endpoint for most individuals infected with HIV (May et al., Dig Dis Sci, 38:1388, 1993).

### Hypothyroidism

Hypothyroidism is a condition in which the thyroid gland does not produce enough thyroid hormone (thyroxine or T4). It is the most common thyroid disorder and symptoms include generalized fatigue, weight gain, thinning (brittle) hair, dry scaly skin, thin nails that break easily, alterations in menses, aching muscles, and slow heart rate. Hypothyroidism also slows the actions of the digestive tract, causing constipation. Rarely, the digestive tract may stop moving entirely. Hypothyroid animals have reduced levels of intestinal alkaline phosphatase (IAP) while hyperthyroid animals dramatically over-expressed IAP (Hodin et al., Surgery, 120:138, 1996; Malo et al., Mol Endocrinol, 18:1941, 2004).

### Colorectal Carcinoma

It has been reported that a correlation exists between the mortality rates for colorectal carcinoma in certain countries and the bacterial and chemical composition of the feces of the different populations (Hill et al., Lancet, 1:95, 1971). Samples from those countries with a higher incidence of the disease had a higher frequency of bacteria that could degrade bile salts. As a result, the stools from people living in these countries also had higher concentrations of neutral and acid sterols derived from the breakdown of cholesterol and bile salts. It is noteworthy that bile acids have been reported to be mutagenic (Watabe and Bernstein, Mutat Res, 158:45, 1985).

More recently, it has been proposed that high-fat, high-protein, and low-fiber diets can increase the risk of large bowel carcinoma (McBurney et al., Nutr Cancer; 10:23, 1987). McBurney *et al.* (1987) proposed that the typical Western diet results in an irregular supply of food in the colon. As the small amount of available carbohydrate in the colon is depleted, fermentation ensues and carcinogens are concentrated into a small colonic mass. There may be enhanced microbial degradation of colonic mucosa. Some microbial species seek alternative substrates (e.g., ferment glycoproteins) and may ultimately cause pre-cancerous lesions in the gastrointestinal tract. The longer this "feast or famine" situation prevails, the more likely it is that the flora and fermentation end-products will promote the progression of malignant disease.

### Obesity

Obese mice have a distinct gut flora compared to normal, lean mice, reflected in a change in the ratio between bacteria from the divisions Bacteroidetes and Firmicutes, which is shifted towards less Bacteroidetes and more Firmicutes in obese mice. The microbes occupying the human gut are also in direct relation to obesity. A shift in the ratio between bacterial divisions Firmicutes and Bacteroidetes can be observed in lean and obese individuals, with a shift towards Firmicutes in obese individuals. The ratio between Firmicutes and Bacteroidetes dynamically reflects the overall weight-condition of an individual, shifting towards Bacteroidetes if an obese individual loses weight (Ley et al., Nature, 444:1022, 2006; Turnbaugh et al., Nature, 444:1027, 2006)

### Rheumatoid Arthritis, Eczema, and Allergy

A large body of work suggests that the intestinal flora plays a role in diseases such as rheumatoid arthritis, eczema, and allergy (Peltonen et al., Br J Rheumatol, 33:638, 1994; Lupp and Finlay, Curr Biol, 15:R235, 2005; Toivanen, Ann Rheum Dis, 62:807, 2003).

### Radiotherapy, Chemotherapy, and Stress

Radiotherapy, chemotherapy, and stress have all been shown to wreck havoc on the gastrointestinal flora (Delia et al., Dig Liver Dis, 34 Suppl 2:S84, 2002; Seal et al., J Dig Dis, 8:143, 2007; Salminen et al., Chemotherapy, 41 Suppl 1:5, 1995; Buret, Am J Pathol, 168:3, 2006).

### Food Poisoning

The digestive system is continuously exposed to damage by bacteria, parasites, fungi, viruses, toxins, and chemicals, including drugs and food additives. Many of these insults are ingested and received by the gastrointestinal tract. Bacteria-related food poisoning is a common, usually mild, but sometimes deadly, illness. The majority of food poisoning cases are caused by *Salmonella typhimurium, Staphylococcus aureus, Clostridium perfringens, Clostridium difficile, Vibrio parahaemolyticus, Vibrio cholerae, Bacillus cereus, Listeria monocytogenes, Yersinia enterocolitica, Campylobacter jejuni, Shigella spp.,* and/or enteropathogenic *Escherichia coli.* These bacteria are commonly found in many raw foods and symptoms of poisoning vary with the bacteria involved. Symptoms may include diarrhea, vomiting, nausea, headache, fever, muscle ache, abdominal cramping, kidney failure, and even death.

Normally a large number of pathogenic bacteria must be present to out-compete the commensal bacteria and cause illness. Therefore, one approach to prevent pathogenic bacteria infection is to increase the numbers of commensal bacteria. The main treatment for food poisoning is rehydration. The subject may need to be admitted to the hospital, but this depends on the severity of the dehydration, response to therapy, and ability to drink fluids without vomiting. Children, in particular, may need close observation.

The following are examples of the practice of the invention. The examples demonstrate that the brush border enzyme, IAP, preserves the normal homeostasis of the gastrointestinal tract microbiota and is useful in inhibiting or treating pathogenic infection (e.g., Salmonellosis) and other human clinical conditions associated with dysregulated intestinal microbiota, including antibiotic-associated illnesses (AAD, CDAD), other gut inflammatory conditions (AIDS), hypothyroidism, colorectal carcinoma, obesity, rheumatoid arthritis, eczema, allergy, radiotherapy treatment, chemotherapy treatment, stress and food poisoning.

### EXAMPLES

The invention is further described in the following examples, which do not limit the scope of the invention described in the claims.

### Example 1: Intestinal Alkaline Phosphatase Enhances the Restoration of Gut Microbiota Lost During Antimicrobial Treatment with Streptomycin

To address the role of intestinal alkaline phosphatase on the gastrointestinal tract flora of mammals, an experiment was performed in which five approximately six week old mice (C57BL/6) of each test group were treated with an antimicrobial drug, streptomycin, in the presence or absence of calf intestinal alkaline phosphatase (cIAP) (Sigma, St. Louis, MO). Both streptomycin (5 mg/ml) and cIAP (50 units/ml) were administered orally with the animal's drinking water.

Individual stool samples of each group were cultured in LB media each day and analyzed for the presence/absence of gut flora. The results are shown in Table 1. The gastrointestinal tract flora of mice before antibiotic treatment was considered as a baseline control for the normal gut flora (present in all samples). The group of mice receiving only cIAP also displayed the normal gut flora phenotype. Both treatment groups received streptomycin for 24 hours. The treatment group receiving only streptomycin had no bacterial growth in their stools 24 hours after discontinuation of the streptomycin treatment. The stools of animals from this treatment group remained sterile for up to nine days and showed signs of the normal gastrointestinal tract flora only at Day 10 of the experiment.

The treatment group receiving streptomycin plus cIAP also showed no growth of gut flora in their stools 24 hours after discontinuation of the streptomycin treatment, indicating the expected antimicrobial effect of streptomycin. However, as soon as 48 hours after stopping the streptomycin treatment, normal levels of gastrointestinal tract flora were reestablished in the stools of these test animals in the presence of cIAP.

Although cIAP did not affect normal gut flora of test animals in the absence of streptomycin, it significantly enhanced the early reestablishment of normal gut flora after discontinuation of the antimicrobial drug treatment when co-administered throughout the treatment period. In addition, although the treated mice had softer stools than normal, none of the mice had diarrhea at any time during the test.

**Table 1. Intestinal alkaline phosphatase (IAP) enhances restoration of gut microbiota lost due to streptomycin antibiotic treatment.**

| | Bacteria in the stool | |
|---|---|---|
| | Streptomycin alone | Streptomycin + IAP |
| Day 1 | Absent | Absent |
| Day 2 | Absent | Present |
| Day 3 | Absent | Present |
| Day 4 | Absent | Present |
| Day 5 | Absent | Present |
| Day 6 | Absent | Present |
| Day 7 | Absent | Present |
| Day 8 | Absent | Present |
| Day 9 | Absent | Present |
| Day 10 | Present | Present |

### Example 2: Intestinal Alkaline Phosphatase Enhances the Restoration of Gut Microbiota Lost During Antimicrobial Treatment with Ampicillin

An experiment was performed in which five mice (C57BL/6) of each test group, approximately 6 weeks old, were treated with 1 mg/ml of the antimicrobial drug ampicillin, in the presence or absence of calf intestinal alkaline phosphatase (clAP) (New England Biolabs, lpswich, MA). Ampicillin was administered orally with the animal's drinking water at a concentration of 1 mg/ml, and 250 units of cIAP were administered twice daily as oral gavage.

Individual stool samples of each group were cultured in LB media each day and analyzed for the presence/absence of the gut flora. The results are shown in Table 2. The gut flora of mice before receiving the antibiotic was considered as a baseline control for normal gastrointestinal tract flora (present in all samples). The group of mice receiving only cIAP also displayed the normal gut flora phenotype. Both treatment groups received ampicillin for 10 days. Both treatment groups receiving ampicillin continued to show bacterial growth 24 hours post treatment initiation and loss of bacterial growth for the rest of the 10 day treatment period, indicating the expected antimicrobial effect of ampicillin. The treatment group receiving only ampicillin had no bacterial growth in their stools until Day 23. The animals showed a normal gut flora only by Day 24 of the experiment. However, the stools of animals from the treatment group receiving ampicillin in combination with cIAP remained sterile for 13 days and showed signs of the normal gastrointestinal tract flora at Day 14 of the experiment.

Although administration of clAP did not affect normal gut flora of test animals in the absence of ampicillin, the treatment significantly reduced the time for restoring the normal gut flora in this experiment (thus, the duration of AAD in this experiment was reduced). In addition, although the treated mice had softer stools than normal, none of the mice had diarrhea at any time during the test.

**Table 2. Intestinal alkaline phosphatase (IAP) enhances restoration of gut microbiota lost due to ampicillin antibiotic treatment.**

| | Bacteria in the stool | |
|---|---|---|
| | Ampicillin alone | Ampicillin + IAP |
| Day 1 | Present | Present |
| Day 2 | Absent | Absent |
| Day 3 | Absent | Absent |
| Day 4 | Absent | Absent |
| Day 5 | Absent | Absent |
| Day 6 | Absent | Absent |
| Day 7 | Absent | Absent |
| Day 8 | Absent | Absent |
| Day 9 | Absent | Absent |
| Day 10 | Absent | Absent |
| Day 11 | Absent | Absent |
| Day 12 | Absent | Absent |
| Day 13 | Absent | Absent |
| Day 14 | Absent | Present |
| Day 15 | Absent | Present |
| Day 16 | Absent | Present |
| Day 17 | Absent | Present |
| Day 18 | Absent | Present |
| Day 19 | Absent | Present |
| Day 20 | Absent | Present |
| Day 21 | Absent | Present |
| Day 22 | Absent | Present |
| Day 23 | Absent | Present |
| Day 24 | Present | Present |

### Example 3: Gram-Negative Aerobic Commensal Bacteria are Absent from the Stools of IAP-Knockout Mice

The effects of IAP on the intestinal flora in wild type (WT) and IAP knockout (KO) mice were investigated. Inactivation of the IAP gene in mice (*Mus musculus* C57BL/6) has been described *(*Narisawa et al., Mol Cell Biol, 23:7525, 2003) and heterozygous mice were obtained from the Burnham Institute for Medical Research (La Jolla, CA). These animals lack the ability to produce IAP, and were subsequently bred at the MGH animal facility to create homozygous IAP-KO, heterozygous, and wild type C57BL/6 (WT) littermates. Confirmation of genotype was performed by PCR analysis (Narisawa et al., Mol Cell Biol, 23:7525, 2003).

Stool samples were plated on a variety of microbiological media, including MacConkey agar, which primarily allows the growth of Gram-negative bacteria. The densities (weight/volume ratio) of the stools of WT and KO mice did not vary significantly, and the protein concentrations per gram stool of these animals were similar. Each experiment was repeated at least five times and similar results were obtained.

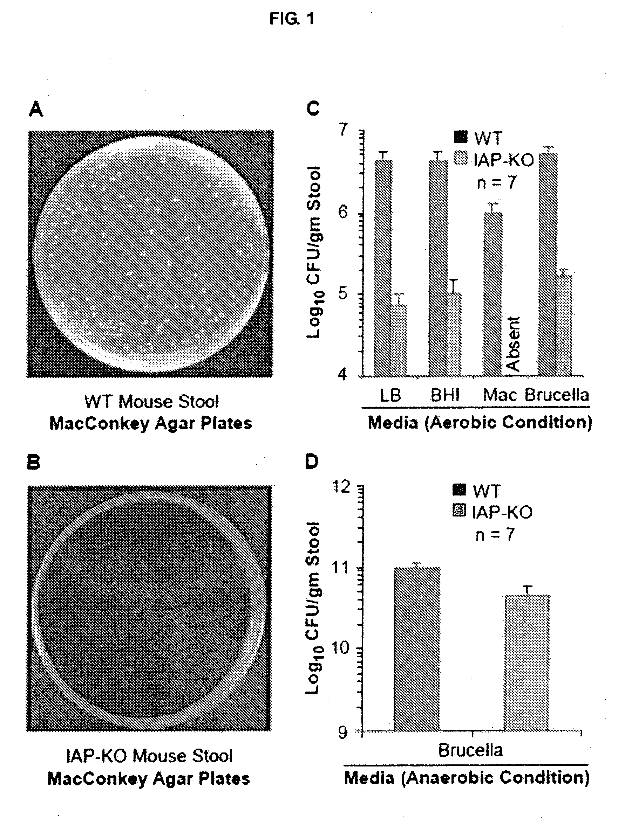

Stool samples from WT animals grew numerous colonies on MacConkey plates (FIG. 1A); however, in dramatic contrast, no commensal bacterial growth was observed on these plates when testing the fecal samples from IAP-KO animals (FIG. 1B). Over 100 fecal cultures from IAP-KO mice were plated and no aerobic Gram-negative flora were observed on MacConkey agar plates. To determine if small amounts of Gram-negative aerobic flora were simply being missed, fecal specimens from 10 KO mice were enriched and subcultured in both Gram-negative enrichment broth and in Luria Bertani (LB) broth. Large volumes of these primary cultures were plated on MacConkey agar and no growth was observed. Most of the aerobic fecal flora isolated from MacConkey plates from WT animals were *E. coli;* however, occasionally, the growth of *Proteus mirabilis* was observed.

To ascertain whether this was a cohort or cage effect, mixed housing experiments were performed in which WT animals were caged with KO littermates. Even when housed together for up to 60 days, KO animals failed to acquire an aerobic Gram-negative flora from their WT littermates as measured by plating of the stool samples on MacConkey plates. The observation that commensal Gram-negative bacteria are absent in the stools of IAP-KO mice suggests that IAP is involved in the maintenance of intestinal microbiota.

### Example 4: Total Number of Aerobic and Anaerobic Commensal Bacteria is Greatly Reduced in the Stools of IAP-KO Mice

Quantitative cultures of WT and IAP-KO stools were performed under aerobic and anaerobic conditions using a variety of rich solid agar media. Individual stool samples from different animals were collected fresh directly in Brain Heart Infusion (BHI) media (200 µl) in microfuge tubes, kept on ice, and weighed. The weight of stool was determined by subtracting the pre-determined weight of tube and media. BHI media was added to each tube obtaining a specific weight:volume ratio (1 mg stool:10 µl BHI). The stool sample was then vortexed to homogenize the sample, followed by serial dilution and plating on LB agar, BHI agar, MacConkey agar, and Brucella (5% horse blood) agar plates.

For the growth of aerobic bacteria, plates were incubated in ambient air for 24 hours at 37°C. For the growth of anaerobic bacteria, samples were plated on the bench and then placed in a table-top anaerobic chamber with CO₂-producing gas packs (Fisher Scientific) for 7 days at 37°C. Anaerobic conditions were rigorously monitored with indicators inside the chamber and typically achieved within 30 minutes in a small chamber holding 10 plates. Colony forming units (CFU) were counted and expressed as CFU/gm of stool +/- SEM. FIG. 1C shows that when plated aerobically on LB, BHI, MacConkey, and Brucella agar plates, the stools of KO mice yielded dramatically fewer commensal bacteria colonies compared to the stools of WT animals. The bacterial counts in LB, BHI, and Brucella plates numbered approximately 3-6 x 10⁶ CFU/gm of stool in the case of WT animals, whereas the stools from IAP-KO mice had bacterial counts of approximately 3 x 10⁵ CFU/gm of stool. Stool culture on the MacConkey media showed approximately 10⁶ Gram-negative bacteria per gram of stool of WT animals; and again, as expected, there was no commensal bacterial growth from the stools of KO animals (see also FIG. 1B).

To quantify commensal anaerobic bacteria, stool samples from WT and KO mice were rapidly plated on Brucella agar plates and incubated at 37°C for 7 days in an anaerobic chamber. Anaerobic bacterial counts in the stools of KO mice were approximately half of the anaerobic bacterial counts in the stools of the WT littermates (4.4 x 10¹⁰ vs. 9.7 x 10¹⁰ CFU/gm of stool, for KO and WT, respectively) (FIG. 1D).

Taken together, these stool culture results demonstrate a generalized decrease in commensal bacteria flora in IAP-KO mice. The most dramatic finding was a complete absence of E. *coli* in the IAP-KO animals. These observations indicate that IAP plays a role in the maintenance of intestinal microbiota.

### Example 5: Terminal Restriction Fragment Length Polymorphism (TRFLP) of 16S rRNA Genes Reveals Differential Intestinal Microbiotal Profile in the Stools of IAP-KO Mice

Fecal bacterial differences between WT and KO mice were further defined using TRFLP analyses of the bacterial 16S rRNA genes. Fecal samples of equal weight were collected from 8 animals in each group (WT and KO). Using the MoBio PowerSoil^{®} soil DNA kit (MoBio Laboratories, Carlsbad, CA), bacterial DNA was extracted from each sample following the manufacturer's protocol. Success of each extraction was determined by measuring DNA concentration in the extraction product with a Spectramax^{®} spectrophotometer (Molecular Devices, Palo Alto CA). PCR was performed to amplify a 500 bp bacterial DNA fragment using primers homologous to conserved regions on the bacterial 16S rRNA gene. The forward primer (16s.8F: 5'-AGAGTTTGTTCMTGGCTCAG-3' (SEQ ID NO:1)) and the reverse primer (16s.530R: 5'-GTATTACCGCGGCTGCTGG-3' (SEQ ID NO:2)) (Kaplan et al., Appl Environ Microbiol, 67:1935, 2001) were fluorescently labeled with a phosphamide dye (D4; GenSet, La Hoya CA) and used in PCR reaction.

PCR reactions (50 µl) were carried out using 1 µl of undiluted DNA extract, 5 µl of 10x Buffer, 3 µl of 10 mM dNTP, 2 µl of 20 mg/ml BSA, 7 µl of 25 mM MgCl₂, 1 µl (10 pmol) of each primer, and 0.3 µl of 5 U/µl TaqGold^{®} DNA polymerase (Applied Biosystems, Foster City, CA). Reaction conditions were: 96°C for 10 minutes; 35 cycles of 94°C for 1 minute, 46.5°C for 1 minute, and 72°C for 2 minutes, followed by a final extension step of 10 minutes at 72°C. All reactions were performed in triplicate and then combined using a MoBio Ultraclean^{®} PCR Cleanup Kit (MoBio Laboratories, Carlsbad, CA) following the manufacturer's protocol. PCR products were quantified using a fluorometer tuned to the labeling dye.

TRFLP was performed following the protocols as previously described (Engelbrektson et al., FEMS Microbiol Ecol, 57 :239, 2006; Blackwood et al., Appl Environ Microbiol, 69:926, 2003). An enzyme digest was performed on 75 ng of cleaned PCR product using one of three restriction endonucleases *Dpn* II, *Hae* III, and *Hpa* II (New England Biolabs, Ipswich, MA). Each 40 µl digestion used 75 ng of DNA, 1 U of enzyme, and 4 µl of buffer. The samples were digested for 4 hours at 37°C and inactivated for 20 minutes at 65°C or 85°C depending on the enzymes inactivation requirement. The digestion products were precipitated by ethanol and resuspended in 20 µl of formamide containing 0.25 µl of CEQ™ 600 base pair DNA standard ladder. Terminal restriction fragment profiles were obtained using a Beckman Coulter CEQ™ 8000 DNA analysis system.

Terminal restriction fragment (TRF) length in nucleotides and TRF peak area were exported from the CEQ™ 8000 system into Microsoft Excel^{®} (Seattle, WA). To standardize the data for comparison between samples, the area under each TRF peak was normalized to total amount of DNA analyzed and expressed as parts per million (ppm). Peaks with an area of less than 10,000 ppm (< 1% of the total for that sample) were excluded from analysis to reduce noise.

Normalized TRFLP data sets were transformed by taking the square root of the area under each TRF peak to de-emphasize large TRF peaks while still taking relative abundance into account (Li et al., J Microbiol Methods, 68: 303, 2007). Transformed data were analyzed by Bray-Curtis similarity and ordinated by multi-dimensional scaling (MDS) using the PRIMER5 statistics package (Primer-E Ltd., Ivybridge, United Kingdom). Significance testing was performed using analysis of similarity (ANOSIM) with PRIMER5.

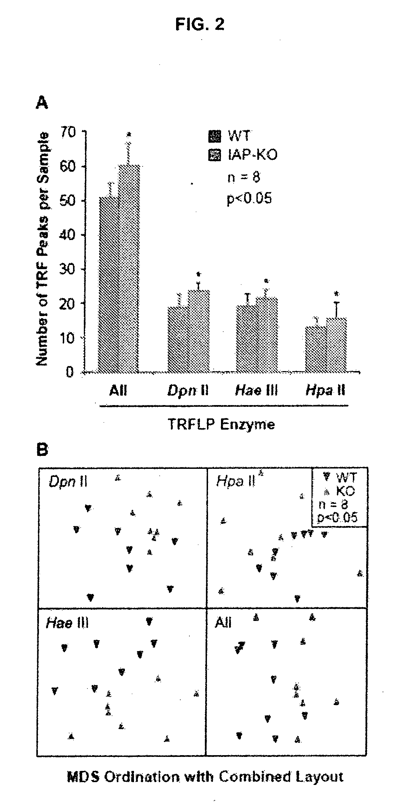

FIG. 2A shows the number of TRF peaks per sample after *Dpn* II, *Hae* III, and *Hpa* II digests as well as TRF peaks from the combination of all 3 restriction digests. There were significantly more TRF peaks in KO samples (p < 0.05), indicating a difference in the types of bacteria present compared to WT. FIG. 2B shows the ordination of *Dpn* II, *Hae* III, and *Hpa* II TRF peaks using multidimensional scaling (MDS). The TRFLP data were similar among the animals within each group (WT and KO) and significantly different between the groups (p ≤ 0.05).

### Example 6: Phylogenetic Analyses Reveal an Altered Profile of Intestinal Microbiota in the Stools of IAP-KO Mice

The differences in TRFLP data indicated phylogenetic changes in fecal bacteria from WT and KO mice. Pools of 10 stool samples from each of WT and KO animals were used to extract bacterial DNA for sequence analysis. The same 500 bp fragment of the 5' end of 16S rRNA gene used in the TRFLP analysis (Example 5) was amplified by PCR. The PCR conditions were: initial denaturation for 2 minutes; 32 cycles of denaturation (94°C for 30 seconds), annealing (45°C for 30 seconds), and extension (72°C for 60 seconds), followed by a final extension step of 5 minutes at 72°C. PCR products were verified by electrophoresis and cloned into a pCR2.1 TA cloning vector following the manufacturer's protocol (Invitrogen, Carlsbad, CA). Transformants were plated on LB-agar plates containing ampicillin (100 µg/ml) and X-gal (40 mg/ml), and incubated overnight at 37°C.

Approximately 1,000 white transformant colonies from each group (WT and KO) were grown at 37°C overnight in 96-well plates each well containing 150 µl of LB-broth with 100 µg/ml ampicillin. Clones from each library were sequenced bidirectionally using M13 forward and reverse primers, resulting in 805 and 877 sequences of 16S rRNA genes from bacteria in WT and KO mice stools, respectively. Sequences were analyzed with the 'Classifier' program developed by Michigan State University (Wang et al., Appl Environ Microbiol, 73:5261, 2007). The program produced the name and number of 16S rRNA gene sequences, and arranged them in taxonomical hierarchy. Percentage of each sequence was calculated using the Microsoft Excel^{®} program. Chi squared analysis was performed to determine statistical significance in the distribution of clones in WT and KO libraries.

A distribution of the bacterial types to the level of family is shown in Table 3 and statistically significant differences (p < 0.05) are highlighted by an asterisk (*). The results indicate that the majority of bacteria in the stools from either group belong to the Bacteroidetes phylum, and there is no significant difference in the relative percentage of Bacteroidetes between WT and KO animals (55% vs. 51%). Firmicutes constitute approximately 23% of the bacterial population in the stools of both WT and KO animals. Interestingly, a higher number of pathogenic Clostridia species was present in the KO stools compared to the stools of WT animals (4.56% vs. 2.36%, KO vs. WT, p < 0.05). For unclassified Firmicutes, the WT stools had greater number than the KO stools (11.8% vs. 8.78%, p < 0.05). About 21% of the bacteria could not be classified in the case of stools of WT animals, whereas the number of unclassified bacteria made up to 25% of the microbiota in the stools of KO mice (p < 0.05). A minimal number (< 1%) of Proteobacteria (aerobic, Gram-negative) were present in the stools of the WT and KO mice.

These phylogenetic data confirm the differences in the number and types of bacteria in the stools of WT and IAP-KO mice and further suggest that IAP functions to regulate the intestinal microbiota. It should be noted that because less than 1,000 clones were sequenced from each library, we could not expect to find statistically significant differences in aerobic, Gram-negative bacteria (e.g., *E. coli*) that were present in relatively low numbers.

The culture, TRFLP, and phylogenetic data together establish a specific role of IAP in the regulation of gut microbiota. While bacterial counts are decreased in the IAP-KO animals, TRF peaks are increased. Some specific bacteria (e.g., Clostridia and Unclassified Bacteria), which are minor in the WT animals, comprise a significant portion of the bacterial population in KO animals. In contrast, some bacterial populations (e.g., Unclassified Firmicutes) that are predominant in the WT animals, are minor in IAP-KO animals. Therefore, IAP-KO animals suffer from decreased numbers of commensal bacteria and a greater variety of pathogenic bacteria in their gastrointestinal tract.

**Table 3. Phylogenetic profile of bacteria in the stools of WT and IAP-KO mice as determined by analyses of 16S rRNA gene sequences.**

| | No. of Sequences | | Percentage (%) of Sequence | |
|---|---|---|---|---|
| | WT | KO | WT | KO |
| domain Bacteria | 805 | 877 | 100 | 100 |
| » phylum Proteobacteria | 6 | 6 | 0.75 | 0.68 |
| » » class Deltaproteobacteria | 2 | 1 | 0.25 | 0.11 |
| » » » unclassified_Deltaproteobacteria | 2 | 1 | 0.25 | 0.11 |
| » » class Epsilonproteobacteria | 2 | 3 | 0.25 | 0.34 |
| » » » order Campylobacterales | 2 | 3 | 0.25 | 0.34 |
| » » » » family Helicobacteraceae | 2 | 3 | 0.25 | 0.34 |
| » » class Betaproteobacteria | 1 | 2 | 0.12 | 0.23 |
| » » » unclassified_Betaproteobacteria | 1 | 2 | 0.12 | 0.23 |
| » » class Gammaproteobacteria | 1 | 0 | 0.12 | 0 |
| » » » order Enterobacteriales | 1 | 0 | 0.12 | 0 |
| » » » » family Enterobacteriaceae | 1 | 0 | 0.12 | 0 |
| » phylum Firmicutes | 187 | 201 | 23.2 | 22.9 |
| » » class "Clostridia" | 19 | 40* | 2.36 | 4.56 |
| » » » order Clostridiales | 19 | 40 | 2.36 | 4.56 |
| » » » » family "Ruminococcaceae" | 1 | 5 | 0.12 | 0.57 |
| » » » » family "Lachnospiraceae" | 9 | 18 | 1.12 | 2.05 |
| » » » » family Incertae Sedis XV | 0 | 1 | 0 | 0.11 |
| » » » » unclassified_Clostridiales | 9 | 16 | 1.12 | 1.82 |
| » » class "Bacilli" | 73 | 84 | 9.07 | 9.58 |
| » » » order "Lactobacillales" | 67 | 76 | 8.32 | 8.67 |
| » » » » family Lactobacillaceae | 1 | 1 | 0.12 | 0.11 |
| » » » » family "Carnobacteriaceae" | 0 | 1 | 0 | 0.11 |
| » » » » unclassified_"Lactobacillales" | 66 | 74 | 8.2 | 8.44 |
| » » » unclassified_"Bacilli" | 6 | 8 | 0.75 | 0.91 |
| » » unclassified_Firmicutes | 95 | 77* | 11.8 | 8.78 |
| » phylum Bacteroidetes | 445 | 450 | 55.3 | 51.3 |
| » » class Bacteroidetes | 289 | 299 | 35.9 | 34.1 |
| » » » order Bacteroidales | 289 | 299 | 35.9 | 34.1 |
| » » » » family Prevotellaceae | 7 | 8 | 0.87 | 0.91 |
| » » » » family Rikenellaceae | 12 | 12 | 1.49 | 1.37 |
| » » » » family Bacteroidaceae | 5 | 9 | 0.62 | 1.03 |
| » » » » family Porphyromonadaceae | 0 | 1 | 0 | 0.11 |
| » » » » unclassified_Bacteroidales | 265 | 269 | 32.9 | 30.7 |
| » » unclassified_Bacteroidetes | 156 | 151 | 19.4 | 17.2 |
| » » unclassified_Bacteria | 167 | 220* | 20.7 | 25.1 |

### Example 7: The Numbers of Stool Bacteria Are Decreased in Hypothyroid Mice and Increased in Hyperthyroid Mice.

In WT mice, IAP (Akp3 isoform) is expressed at postnatal Day 15 (Narisawa et al., Am J Physiol Gastrointest Liver Physiol, 293:G1068, 2007), while IAP-KO mice lack this enzyme for their entire life. However, a variety of pathophysiological conditions can lead to altered IAP expression in adult WT animals, which may affect their intestinal flora. Accordingly, stool samples of hypothyroid and hyperthyroid mice were examined, since IAP is known to be silenced in hypothyroid animals and dramatically over-expressed in hyperthyroid animals *(*Hodin et al., Surgery, 120:138, 1996; Malo et al., Mol Endocrinol, 18:1941, 2004). For 32 weeks, groups of mice (n = 5) were given water containing 0.05% propylthiouracil (PTU) in order to induce hypothyroidism by inhibiting both thyroid hormone biosynthesis and peripheral T4 deiodination (Cooper et al., Biochem Pharmacol, 33:3391, 1984). After 6 weeks of PTU treatment, one group received a daily i.p. injection of T3 (30)µg per 100 gm body weight, hyperthyroid) and the other group received a daily i.p. injection of normal saline (0.9% NaCl, hypothyroid) for 26 weeks (Hodin et al., Surgery, 120:138, 1996).

IAP activity was determined following the protocol as described by Baykov et al. (Anal Biochem, 171:266, 1988). Briefly, individual stool samples were homogenized in water (10 mg/100 µl, centrifuged and supernatant was collected. Then 25 µl of the stool supernatant (or aqueous cIAP solution) were mixed with 175 µl phosphatase assay reagent containing 5 mM of p-nitrophenyl phosphate (pNPP) followed by determining optical density at 405 nm after a specific time period when the samples usually turned yellow due to release of p-nitrophenol. Protein concentrations of stool supernatants were measured using a kit from Bio-Rad Laboratories (Hercules, CA). The specific activity of the enzyme, expressed as pmole pNPP hydrolyzed per minute per µg of protein, and CFU per gm of stool +/- SEM were determined and the results are shown in FIG. 3. As expected, IAP activity is lower in hypothyroid animals (Weeks 6-32, FIG. 3A) as compared to the euthyroid animals (Week 0, FIG. 3A). The animals receiving T3 injections showed increased IAP activity in their stools from Weeks 12-32, and the longer the animals received T3 injections, the higher was the IAP activity. At Week 32 the IAP activity in hyperthyroid animals was approximately 7-fold higher compared to euthyroid animals and 15-fold higher compared to hypothyroid animals. The number of bacteria in the stools of hypothyroid mice (low IAP levels) gradually decreased in a time-dependent manner compared to the euthyroid mice (FIGs. 3B, 3C, and 3D). On the other hand the fecal bacterial counts in hyperthyroid animals continued to gradually increase, being the highest at Week 32 when the experiment was terminated (FIGs. 3B, 3C, and 3D).

FIG. 3B shows that the number of total bacteria grown aerobically on LB agar is dramatically reduced in the case of hypothyroid animals (Weeks 6-32) compared to euthyroid animals (Week 0), and this decrement is directly related to the duration of hypothyroidism; the longer the animals are in a hypothyroid state, the greater is the decrement of fecal bacteria. At Week 32, hypothyroid animals had nearly 2 log less commensal bacteria in their stools compared to the WT animals. In contrast, the hyperthyroid animals had approximately 1 and 3 log more commensal bacteria compared to WT and hypothyroid animals, respectively. FIG. 3C shows that Gram-negative bacteria (E. *coli)* nearly disappear from the stools of hypothyroid animals at Week 32, similar to what was observed in IAP-KO mice (see FIGs. 1B and 1C). On the other hand, hyperthyroid animals show dramatic overgrowth of Gram-negative bacteria *(E. coli)* at Weeks 24-32 compared to the WT, hypothyroid, and IAP-KO animals (9 x 10⁶, 3 x 10⁵, 2 x 10⁴, and 0 CFU/gm of stool for hyperthyroid, WT, hypothyroid, and IAP-KO, respectively; also see FIGs. 1B and 1C).

The hypothyroid animals have dramatically fewer anaerobic bacteria in their stools compared to the WT and hyperthyroid mice (FIG. 3D). The decrement of anaerobic bacteria is also dependent on the duration of hypothyroidism status; the longer the animals suffer from hypothyroidism, the lower is the number of fecal anaerobes. Conversely, the number of anaerobic fecal bacteria gradually increases with increased duration of hyperthyroidism. At Week 32 hyperthyroid animals have 4 and 5 log more fecal anaerobes in their stools compared to WT and hypothyroid animals, respectively. The maximum growth of both aerobes and anaerobes in hyperthyroid animals occurs when the endogenous IAP expression is also the highest, which supports a role of IAP in promoting growth of commensal bacteria *in vivo.* These results further suggest that a hypothyroid subject be administered IAP to increase the number of commensal bacteria in their gastrointestinal tract.

### Example 8: Commensal E. coli Fails to Colonize IAP-KO Mice

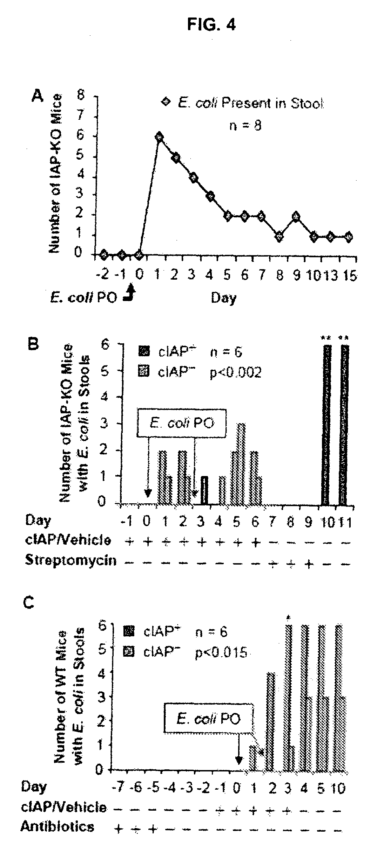

*E. coli* was introduced to IAP-KO animals by oral gavage and its ability to colonize the gastrointestinal tract was monitored for a period 15 days. A commensal E. *coli* was isolated from the stool of a WT mouse and a spontaneous streptomycin-resistant *E. coli* was isolated on MacConkey agar plate containing streptomycin (100 µg/ml). Eight KO mice were fed this streptomycin-resistant E. *coli* (6.0 x10⁵ CFU) in PBS, and animals were followed daily by collecting and plating fresh fecal samples. The inoculated commensal organism was initially present in only six of eight animals after one day of inoculation, and subsequently eliminated by all but one animal after 15 days, when the experiment was terminated (FIG. 4A). These results indicate a probable hostile intraluminal environment for commensal bacteria in IAP-KO animals.

### Example 9: Oral Supplementation of IAP Favors the Survival of Commensal E. coli in IAP-KO Mice

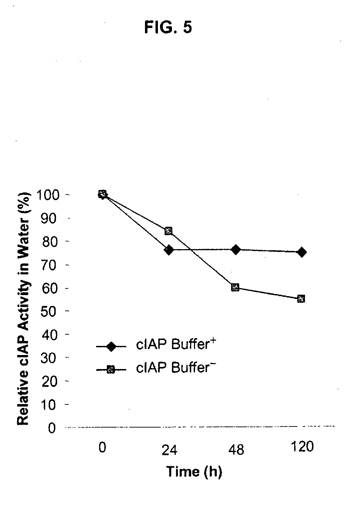

Investigations were performed to determine if the hostile intraluminal environment for *E. coli* in IAP-KO mice, as described in the Examples above, could be reversed by oral supplementation of IAP. Prior to starting the experiment, the activity and stability of calf IAP (cIAP) in drinking water was assayed in addition to IAP activity in the stools of five animals receiving cIAP in their water. Two units of cIAP (New England Biolabs, Ipswich, MA) were dissolved in 1 ml autoclaved tap water in the presence or absence of cIAP buffer, and clAP activity was determined at specific time intervals *(*Baykov et al., Anal Biochem, 171:266, 1988). cIAP is reasonably stable in tap water and approximately 80% of cIAP enzymatic activity remains after 24 hours at room temperature without any added cIAP buffer (FIG. 5). Animals received cIAP in autoclaved drinking water, which was replaced every day to avoid using cIAP buffer because the buffer is basic (pH 7.9) and has high concentrations of salts (100 mM NaCl, 50 mM Tris-HCl, 10 mM MgCl₂, and 1 mM DTT), which might affect background bacterial growth and mask the effects of cIAP.

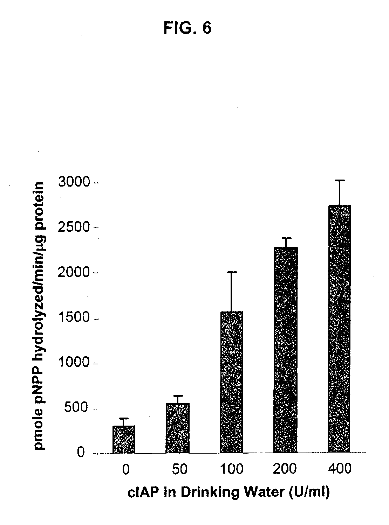

Fecal IAP enzyme activity increased in a dose-dependent fashion in mice given water with added cIAP (FIG. 6). A group of mice (n = 5) were allowed to drink water containing a specific amount of cIAP for 24 hours and the amount of IAP activity in their stools was assayed. Specific activity of IAP is expressed as average pmole pNPP hydrolyzed/minute/mg protein +/- SEM. The results show that the IAP activity in a stool sample is dose-dependent; the greater the amount of IAP in the drinking water, the higher the activity in the stool sample. This indicates that oral administration of IAP in drinking water is an effective route of delivery to increase intestinal luminal concentration of IAP.

Two groups of IAP-KO animals (n = 6) were put on drinking water containing cIAP or 'vehicle for cIAP.' On Day 2, animals were gavaged with 20,000 CFU of streptomycin-resistant *E. coli,* which is a commensal bacterium isolated from the stool of a WT mouse, and the presence of streptomycin-resistant *E. coli* in the stools of these animals was closely monitored. As only a few animals carried *E. coli* in their stools (FIG. 4B), the animals were gavaged with another dose of 50,000 CFU of *E. coli* on Day 5, however, again only a few animals shed *E. coli* in their stools. On Day 8, most of the animals of either group carried no *E. coli* in their stools. Supplementation of cIAP was stopped and the animals were treated with streptomycin for 3 days, which should kill most of the native flora, but would allow proliferation of the surviving streptomycin-resistant E. *coli.* All animals of the group receiving cIAP had a very high number of *E*. *coli* (1-7x10⁵ CFU/gm of stool) in their stools after streptomycin treatment (Day 12), whereas animals receiving no cIAP had no *E. coli* in their stools. These data illustrate that *E. coli* can survive in IAP-KO mice supplemented with oral cIAP, indicating a vital role of IAP in the maintenance of commensal bacteria and an application of IAP as an adjuvant to probiotics.

### Example 10: Temporal Enhancement of Colonization of E. coli in WT Mice Supplemented with IAP

To determine the temporal effects of IAP on the restoration of commensal gut flora, groups of WT animals (n = 6) were treated with streptomycin (5 mg/ml) and ciprofloxacin (0.5 mg/ml) for three days followed by oral gavage of small doses (20,000 CFU) of streptomycin-resistant E. *coli* for two alternate days (FIG. 4C; Day 0 and Day 2) in the presence or absence of cIAP. The results (FIG. 4C) show that oral supplementation of cIAP causes statistically significant (p < 0.015) temporal enhancement of colonization of E. *coli* suggesting a role of IAP in the maintenance of gastrointestinal tract flora.

Taken together, these *in vivo* effects of orally administered IAP on commensal E. *coli* indicate a regulatory role of IAP in the maintenance of normal gastrointestinal tract flora.

### Example 11: Oral Administration of the IAP Protein Enhances Restoration of the Commensal Gut Flora Lost in Antibiotic-Treated WT Mice

The experiments described above demonstrate that IAP enhances "uptake" of refed enteric bacteria after antimicrobial eradication. The ability of exogenous IAP to induce restoration of endogenous enteric flora was examined to study its therapeutic use in treating disorders such as antibiotic-associated diarrhea and *C*. *difficile* colitis. Accordingly, experiments were performed in which groups of WT mice (n = 10) were treated orally with the antibiotic streptomycin in the presence or absence of cIAP. Streptomycin (5 mg/ml) and cIAP (200 U/ml for cIAP⁺ Group, n = 5) or 'vehicle for cIAP' (20 µl/ml 'vehicle for cIAP' (50 mM KCl, 10 mM Tris-HCl (pH 8.2), 1 mM MgCl₂, 0.1 mM ZnCl₂, and 50% glycerol) in drinking water for control cIAP⁻ Group, n = 5) were administered in the animals' drinking water and fecal cultures were performed. Because *E. coli* was identified as the most prevalent Gram-negative aerobic bacterium in the WT mouse stool (see Example 3), this commensal organism was followed.

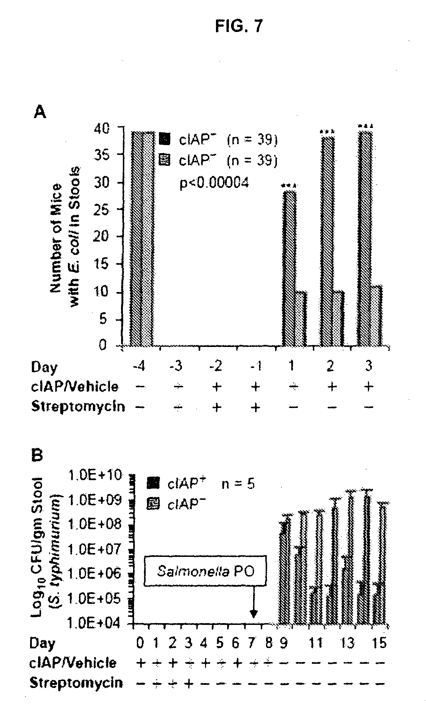

All animals had fecal cultures positive for *E. coli* at the outset of the experiment (FIG. 7A) at levels comparable to those shown in FIG. 1. Individual stool samples were cultured each day on MacConkey agar media. Streptomycin was administered for three days in all animals, which caused an elimination of aerobic Gram-negative organisms in all animals within 24 hours. After discontinuation of antibiotic treatment, the time of appearance (Day) of Gram-negative bacteria (mostly *E. coli,* occasionally *P. mirabilis)* in stool samples was recorded for each animal. The experiment was repeated six times, which raised the total number of animals in each group to 39 (n = 39), however, duration of each experiment varied (7-35 days). Data was compiled from all six experiments and the number of animals with *E. coli* in each group on a specific day was recorded.

The data for 7 days (Day -4 to Day 3), at the end of which all animals of the cIAP⁺ Group showed *E. coli* in their stools, are shown in FIG. 7A. It is evident that at a given point of time (Day), the number of animals with commensal Gram-negative bacteria in the cIAP⁺ Group was much higher than the number of animals with Gram-negative bacteria in the cIAP⁻ Group, and the difference was statistically significant for all time-points (p < 0.00004) as determined by Fisher's Exact Test. For other time points (beyond Day 3, not shown), although the combined total number of animals in each group varied from 29-34, the difference at any time-point was always statistically significant (p < 0.000005). These data suggest that IAP can be used as a therapeutic agent to rapidly restore the commensal gastrointestinal tract microflora in the setting of antibiotic treatment.

### Example 12: IAP Inhibits the Growth of Salmonella typhimurium in vivo

Restoration or maintenance of the normal gastrointestinal tract flora by IAP was postulated to inhibit infection by pathogenic bacteria because pathogenic bacteria would likely face competition for 'food and shelter' (e.g., nutrition, epithelial attachment sites, space, etc.) and antibacterial factors produced by commensal organisms. Accordingly, groups of WT mice (n = 5) were treated with streptomycin (5 mg/ml) for 3 days, followed by oral gavage of 500,000 CFU of streptomycin-resistant *S*. *typhimurium* in the presence of cIAP (200 U/ml for cIAP⁺ Group) or absence of cIAP (20 µl/ml 'vehicle for cIAP' (50 mM KCl, 10 mM Tris-HCl (pH 8.2), 1 mM MgCl₂, 0.1 mM ZnCl₂, and 50% glycerol) in drinking water for control cIAP' Group). The presence of S. *typhimurium* was monitored in the stools of these animals by plating stools on Hektoen plates containing streptomycin (100 µg/ml). The results (FIG. 7B) show that the growth of pathogenic *S. typhimurium* was dramatically inhibited in animals receiving cIAP in the drinking water. Compared to the cIAP⁻ Group, the *Salmonella* count is approximately 4 log less in the cIAP⁺ Group (p<0.001) and strongly indicates that IAP can inhibit/treat pathogenic infections.

## Claims

1. Alkaline phosphatase for use in reducing one or more symptoms of *Clostridium difficile-*associated disease (CDAD) in a subject, wherein the alkaline phosphatase is used in an amount effective to increase the number of commensal bacteria, decrease the number of pathogenic bacteria, or increase the number of commensal bacteria and decrease the number of pathogenic bacteria in the subject's gastrointestinal tract, thereby reducing one or more symptoms of CDAD.

2. Alkaline phosphatase for the use of claim 1, wherein the alkaline phosphatase is an intestinal alkaline phosphatase.

3. Alkaline phosphatase for the use of claim 1, wherein the alkaline phosphatase is administered by oral, intravenous, or rectal administration.

4. Alkaline phosphatase for the use of claim 1, wherein the alkaline phosphatase is administered at or at about the same time as a diagnosis of aberrant levels of gastrointestinal tract flora.

5. Alkaline phosphatase for the use of claim 1, wherein the alkaline phosphatase is administered before an aberrant level of gastrointestinal tract flora is expected to occur.

6. Alkaline phosphatase for the use of claim 1, wherein the alkaline phosphatase is administered, before, at, or at about the same time as an antibiotic is administered.

7. Alkaline phosphatase for the use of claim 1, wherein the amount of alkaline phosphatase comprises 1 to 10,000 units per kilogram body weight.

8. Alkaline phosphatase for the use of claim 2, wherein the intestinal alkaline phosphatase is a calf intestinal alkaline phosphatase.

9. Alkaline phosphatase for the use for the use of claim 2, wherein the intestinal alkaline phosphatase is a human intestinal alkaline phosphatase.

10. Alkaline phosphatase for the use of claim 1, wherein the alkaline phosphatase is administered by oral administration.

11. Alkaline phosphatase for the use of claim 1, wherein the alkaline phosphatase is administered at or at about the same time as an antibiotic is administered.

12. Alkaline phosphatase for the use of claim 1, wherein the subject is a human.

13. Alkaline phosphatase for use in reducing one or more symptoms of antibiotic-associated diarrhea (AAD) in a subject, wherein the alkaline phosphatase is used in an amount effective to increase the number of commensal bacteria, decrease the number of pathogenic bacteria, or increase the number of commensal bacteria and decrease the number of pathogenic bacteria in the subject's gastrointestinal tract, thereby reducing one or more symptoms of AAD, wherein the pathogenic bacteria are selected from the group consisting of *Actinomyces israelii, Bacillus anthracis, Bacillus brevis, Bacillus cereus, Bacillus fusiformis, Bacillus licheniformis, Bacillus megaterium, Bacillus mycoides, Bacillus stearothermophilus, Bacillus subtilis, Clostridium botulinum, Clostridium difficile, Clostridium perfringens, Clostridium tetani, Corynebacterium diphtheriae, Corynebacterium fusiforme, Enterococcus avium, Enterococcus durans, Enterococcus faecalis, Enterococcus faecium, Enterococcus galllinarum, Enterococcus maloratus, Gardnerella vaginalis, Lactobacillus acidophilus, Lactobacillus casei, Lactococcus lactis, Listeria monocytogenes, Micrococcus luteus, Mycobacterium avium, Mycobacterium bovis, Mycobacterium diphtheriae, Mycobacterium intracellulare, Mycobacterium leprae, Mycobacterium lepraemurium, Mycobacterium phlei, Mycobacterium smegmatis, Mycobacterium tuberculosis, Mycoplasma fermentans, Mycoplasma genitalium, Mycoplasma hominis, Mycoplasma penetrans, Mycoplasma pneumonia, Peptostreptococcus, Rothia dentocariosa, Staphylococcus aureus, Staphylococcus epidermidis, Streptococcus agalactiae, Str-eptococcus avium, Streptococcus bovis, Streptococcus cricetus, Streptococcus faceium, Streptococcus faecalis, Streptococcus ferus, Streptococcus gallinarum, Streptococcus lactis, Streptococcus mitior, Streptococcus mitis, Streptococcus mutans, Streptococcus oralis, Streptococcus, pneumoniae, Streptococcus pyogenes, Streptococcus rattus, Streptococcus salivarius, Streptococcus sanguis,* and *Streptococcus sobrinus.*

14. Alkaline phosphatase for the use of claim 13, wherein the alkaline phosphatase is administered before, at, or at about the same time as an antibiotic is administered.

15. Alkaline phosphatase for the use of claim 13, wherein the alkaline phosphatase is administered by oral administration.

## Patentansprüche

1. Alkalische Phosphatase zur Verwendung zum Vermindern von einem oder mehr Symptomen von *Clostridium difficile-*assoziierten Erkrankungen (CDAD) in einem Patienten, wobei die alkalische Phosphatase in einer Menge benutzt wird, die wirksam ist, um die Anzahl an kommensalen Bakterien zu erhöhen, die Anzahl an pathogenen Bakterien zu verringern, oder um die Anzahl an kommensalen Bakterien zu erhöhen und die Anzahl an pathogenen Bakterien zu verringern, im Magen-Darm-Trakt des Subjekts, wobei eines oder mehr Symptome von CDAD vermindert werden.

2. Alkalische Phosphatase zur Verwendung nach Anspruch 1, wobei die alkalische Phosphatase eine intestinale alkalische Phosphatase ist.

3. Alkalische Phosphatase zur Verwendung nach Anspruch 1, wobei die alkalische Phosphatase durch orale, intravenöse oder rectale Gabe verabreicht wird.

4. Alkalische Phosphatase zur Verwendung nach Anspruch 1, wobei die alkalische Phosphatase zur oder ungefähr zur selben Zeit verbreicht wird wie die Diagnose von abweichenden Niveaus hinsichtlich der Flora des Magen-Darm-Trakts.

5. Alkalische Phosphatase zur Verwendung nach Anspruch 1, wobei die alkalische Phosphatase verbreicht wird bevor ein ewartetes, abweichendes Niveau hinsichtlich der Flora des Magen-Darm-Trakts eintritt.

6. Alkalische Phosphatase zur Verwendung nach Anspruch 1, wobei die alkalische Phosphatase verabreicht wird bevor, zur oder ungefähr zur selben Zeit wie ein Antibiotikum verabreicht wird.

7. Alkalische Phosphatase zur Verwendung nach Anspruch 1, wobei die Menge an alkalischer Phosphatase 1 bis 10000 Einheiten pro Kilogramm Körpergewicht umfasst.

8. Alkalische Phosphatase zur Verwendung nach Anspruch 2, wobei die intestinale alkalische Phosphatase eine intestinale alkalische Phosphatase aus einem Kalb ist.

9. Alkalische Phosphatase zur Verwendung nach Anspruch 2, wobei die intestinale alkalische Phosphatase eine humane intestinale alkalische Phosphatase ist.

10. Alkalische Phosphatase zur Verwendung nach Anspruch 1, wobei die alkalische Phosphatase durch orale Gabe verabreicht wird.

11. Alkalische Phosphatase zur Verwendung nach Anspruch 1, wobei die alkalische Phosphatase verabreicht wird zur oder ungefähr zur selben Zeit wie ein Antibiotikum verabreicht wird.

12. Alkalische Phosphatase zur Verwendung nach Anspruch 1, wobei der Patient ein Mensch ist.

13. Alkalische Phosphatase zur Verwendung zum Vermindern von einem oder mehr Symptomen von Antibiotika-assoziierte Diarrhö (AAD) in einem Subjekt, wobei die alkalische Phosphatase in einer Menge benutzt wird, die wirksam ist, um die Anzahl an kommensalen Bakterien zu erhöhen, die Anzahl an pathogenen Bakterien zu verringern, oder um die Anzahl an an kommensalen Bakterien zu erhöhen und die Anzahl an pathogenen Bakterien zu verringern im Magen-Darm-Trakt des Patienten, wobei eines oder mehr Symptome von AAD vermindert werden, wobei die pathogenen Bakterien ausgewählt sind aus der Gruppe, umfassend: *Actinomyces israelii, Bacillus anthracis, Bacillus brevis, Bacillus cereus, Bacillus fusiformis, Bacillus licheniformis, Bacillus megaterium, Bacillus mycoides, Bacillus stearothermophilus, Bacillus subtilis, Clostridium botulinum, Clostridium difficile, Clostridium perfringens, Clostridium tetani, Corynebacterium diphteriae, Corynebacterium fusiforme, Enterococcus avium, Enterococcus durans, Enterococcus faecalis, Enterococcus faecium, Enterococcus gallinarum, Enterococcus maloratus, Gardnerella vaginalis, Lactobacillus acidophilus, Lactobacillus casei, Lactobacillus lactis, Listeria monocytogenes, Micrococcus luteus, Mycobacterium avium, Mycobacterium bovis, Mycobacterium diphteriae, Mycobacterium intracellulare, Mycobacterium leprae, Mycobacterium lepraemurium, Mycobacterium phlei, Mycobacterium smegmatis, Mycobacterium tuberculosis, Mycoplasma fermentans, Mycoplasma genitalium, Mycoplasma hominis, Mycoplasma penetrans, Mycoplasma pneumonia, Peptostreptococcus, Rothia dentocariosa Staphylococcus aureus, Staphylococcus epidermidis, Streptococcus agalactiae, Streptococcus avium, Streptococcus bovis, Streptococcus cricetus, Streptococcus faecium, Streptococcus faecalis, Streptococcus ferus, Streptococcus gallinarum, Streptococcus lactis, Streptococcus mitior, Streptococcus mitis, Streptococcus mutans, Streptococcus oralis, Streptococcus pneumoniae, Streptococcus pyogenes, Streptococcus rattus, Streptococcus salivarius, Streptococcus sanguis,* and *Streptococcus sobrinus.*

14. Alkalische Phosphatase zur Verwendung nach Anspruch 13, wobei die alkalische Phosphatase verabreicht wird bevor, zur oder ungefähr zur selben Zeit wie ein Antibiotikum verabreicht wird.

15. Alkalische Phosphatase zur Verwendung nach Anspruch 1, wobei die alkalische Phosphatase durch orale Gabe verabreicht wird.

## Revendications

1. Phosphatase alcaline pour utilisation dans la réduction d'un ou plusieurs symptômes de maladie associée à *Clostridium difficile* (CDAD) chez un sujet, la phosphatase alcaline étant utilisée en une quantité efficace pour augmenter le nombre de bactéries commensales, diminuer le nombre de bactéries pathogènes, ou augmenter le nombre de bactéries commensales et diminuer le nombre de bactéries pathogènes dans le tube digestif du sujet, de manière à réduire un ou plusieurs symptômes de CDAD.

2. Phosphatase alcaline pour l'utilisation de la revendication 1, la phosphatase alcaline étant une phosphatase alcaline intestinale.

3. Phosphatase alcaline pour l'utilisation de la revendication 1, la phosphatase alcaline étant administrée par administration orale, intraveineuse, ou rectale.

4. Phosphatase alcaline pour l'utilisation de la revendication 1, la phosphatase alcaline étant administrée à ou environ en même temps qu'un diagnostic de taux aberrants de flore dans le tube digestif.

5. Phosphatase alcaline pour l'utilisation de la revendication 1, la phosphatase alcaline étant administrée avant qu'il soit attendu qu'un taux aberrant de flore dans le tube digestif survienne.

6. Phosphatase alcaline pour l'utilisation de la revendication 1, la phosphatase alcaline étant administrée, avant, à, ou environ en même temps que l'administration d'un antibiotique.

7. Phosphatase alcaline pour l'utilisation de la revendication 1, la quantité de phosphatase alcaline étant de 1 à 10 000 unités par kilogramme de poids corporel.

8. Phosphatase alcaline pour l'utilisation de la revendication 2, la phosphatase alcaline intestinale étant une phosphatase alcaline intestinale de veau.

9. Phosphatase alcaline pour l'utilisation de la revendication 2, la phosphatase alcaline intestinale étant une phosphatase alcaline intestinale humaine.

10. Phosphatase alcaline pour l'utilisation de la revendication 1, la phosphatase alcaline étant administrée par administration orale.

11. Phosphatase alcaline pour l'utilisation de la revendication 1, la phosphatase alcaline étant administrée en même temps ou environ en même temps qu'un antibiotique est administré.

12. Phosphatase alcaline pour l'utilisation de la revendication 1, le sujet étant un humain.

13. Phosphatase alcaline pour utilisation dans la réduction d'un ou plusieurs symptômes de diarrhée associée aux antibiotiques (AAD) chez un sujet, la phosphatase alcaline étant utilisée en une quantité efficace pour augmenter le nombre de bactéries commensales, diminuer le nombre de bactéries pathogènes, ou augmenter le nombre de bactéries commensales et diminuer le nombre de bactéries pathogènes dans le tube digestif du sujet, de manière à réduire un ou plusieurs symptômes d'AAD, les bactéries pathogènes étant choisies dans le groupe constitué *d'Actinomyces israelii, Bacillus anthracis, Bacillus brevis, Bacillus cereus*, *Bacillus fusiformis, Bacillus licheniformis, Bacillus megaterium, Bacillus mycoides, Bacillus stearothermophilus, Bacillus subtilis, Clostridium botulinum, Clostridium difficile, Clostridium perfringens, Clostridium tetani, Corynebacterium diphtheriae, Corynebacterium fusiforme, Enterococcus avium, Enterococcus durans, Enterococcus faecalis, Enterococcus faecium, Enterococcus galllinarum, Enterococcus maloratus, Gardnerella vaginalis, Lactobacillus acidophilus, Lactobacillus casei, Lactococcus lactis, Listeria monocytogenes, Micrococcus lutes, Mycobacterium avium, Mycobacterium bovis, Mycobacterium diphtheriae, Mycobacterium intracellulare, Mycobacterium leprae, Mycobacterium lepraemurium, Mycobacterium phlei, Mycobacterium smegmatis, Mycobacterium tuberculosis, Mycoplasma fermentans, Mycoplasma genitalium, Mycoplasma hominis, Mycoplasma penetrans, Mycoplasma pneumonia, Peptostreptococcus, Rothia dentocariosa, Staphylococcus aureus, Staphylococcus epidermidis, Streptococcus agalactiae, Streptococcus avium, Streptococcus bovis, Streptococcus cricetus, Streptococcus faceium, Streptococcus faecalis, Streptococcus ferus, Streptococcus gallinarum, Streptococcus lactis, Streptococcus mitior, Streptococcus mitis*, *Streptococcus mutans, Streptococcus oralis, Streptococcus pneumoniae, Streptococcus pyogenes, Streptococcus rattus*, *Streptococcus salivarius, Streptococcus sanguins,* et *Streptococcus sobrinus.*

14. Phosphatase alcaline pour l'utilisation de la revendication 13, la phosphatase alcaline étant administrée avant, en même temps, ou environ en même temps qu'un antibiotique est administré.

15. Phosphatase alcaline pour l'utilisation de la revendication 13, la phosphatase alcaline étant administrée par administration orale.
